# EUROPÄISCHE PATENTSCHRIFT

(11) **EP 1 287 839 B1**
(45) Veröffentlichungstag und Bekanntmachung des Hinweises auf die Patenterteilung: **07.03.2007**
(21) Anmeldenummer: 02019581.4
(22) Anmeldetag: 02.09.2002
(51) Int. Cl.: A61M 1/36, G01B 7/00

(54) **Messvorrichtung und -verfahren zur Bestimmung von Parametern medizinischer Flüssigkeiten**
Measurement device and method for determining medical fluid parameters
Dispositif et méthode de mesure pour déterminer des paramètres de fluides médicaux

(30) Priorität: 03.09.2001 DE 10143137
(43) Veröffentlichungstag der Anmeldung: 05.03.2003
(73) Patentinhaber: Fresenius Medical Care Deutschland GmbH, 61352 Bad Homburg (DE)
(72) Erfinder: Metzner, Klaus, 61381 Friedrichsdorf (DE)
(74) Vertreter: Laufhütte, Dieter

(56) Entgegenhaltungen:
- AT-B- 407 303
- DE-A- 19 605 652
- DE-A- 19 746 199
- DE-A- 19 837 667
- US-A- 6 101 406

## Beschreibung

Die Erfindung betrifft eine Meßvorrichtung zur Bestimmung von Parametern medizinischer Flüssigkeiten mit den Merkmalen des Oberbegriffes des Anspruches 1, ein Verfahren zur Bestimmung von Parametern medizinischer Flüssigkeiten mit den Merkmalen des Oberbegriffes des Anspruches 9 und ein Verfahren zur Kalibrierung einer Meßvorrichtung zur Bestimmung von Parametern medizinischer Flüssigkeiten.

Zum Beispiel bei der Dialysebehandlung ist es hilfreich, daß in dem extrakorporalen Blutkreislauf während der Behandlung die Bluttemperatur, das Blutvolumen und/oder der Luftanteil bestimmt werden.

Dazu wird z. B. das Blut während der Dialysebehandlung im extrakorporalen Kreislauf durch eine Disposable-Kassette geleitet, wie es in DE 198 37 667 A1 beschrieben ist. Eine solche Disposable-Kassette besteht aus einem Plastikteil, das wiederum aus zwei weiteren Teilen besteht. In ein Hartteil sind Kanäle und Volumina eingelassen. Dieses Hartteil ist mit einer flexiblen Folie zur Abdeckung der Kanäle und Volumina abgeschlossen. Die Disposable-Kassette wird in eine spezielle Aufnahmekammer, z. B. an einer Dialysemaschine, eingebracht. Diese Kammer kann z. B. mit Hilfe einer schwenkbaren Tür geöffnet werden. Die Disposable-Kassette wird in die Kammer eingelegt, wobei der flexiblen Folie ein entsprechendes Gegenstück an der Maschine gegenüberliegt, damit die Kassette mit Hilfe von maschinenseitigen Aktoren und Sensoren betrieben werden kann. Die Tür wird geschlossen und dient dabei als Widerlager. In der Kammer können Sensoren zur Bestimmung verschiedener Parameter des Blutes während der Dialysebehandlung vorgesehen sein, z. B. ein Temperatursensor oder ein Drucksensor.

Weiterhin kann eine Lichtschranke vorgesehen sein, um festzustellen, ob Blut durch die Kanäle strömt. Mit Hilfe einer Ultraschallmeßeinheit kann eine Laufzeitmessung durch eine Meßkammer in der Disposable-Kassette durchgeführt werden, die eine Bestimmung der Blutdichte, der Konzentration und/oder des Hämatokritwertes zuläßt.

Um aus der Laufzeit präzise den gewünschten Parameter bestimmen zu können, muß die Ultraschallaufstrecke möglichst genau bekannt sein. Bei der bekannten Lösung der DE 198 37 667 A1 wird deswegen die Disposable-Kassette mit Hilfe von Abstandsbolzen und Verschraubungen in der Aufnahmekammer gehalten, so daß sich ein präziser Abstand ergibt.

Eine solche Konstruktion ist jedoch aufwendig und erfordert eine längere Vorbereitung der Dialysebehandlung. Durch die Abstandsbolzen ist die Anordnung gegen Verschmutzung und Verschleiß empfindlich. Die während einer Behandlung, die mehrere Stunden dauern kann, auf die Kassette wirkenden Andruckkräfte können z. B. zu einer plastischen Verformung der Kassette und damit des Abstandes führen. Andererseits sind Durchbrüche durch die Kassette und die Folie notwendig, um die Abstandsbolzen fixieren zu können.

Ausgehend von diesem Stand der Technik ist es die Aufgabe der vorliegenden Erfindung, eine Vorrichtung und ein Verfahren anzugeben, mit denen die genaue Bestimmung eines Parameters einer medizinischen Flüssigkeit möglich ist, dessen Wert von der Ausdehnung einer Meßkammer abhängt, wobei die Meßvorrichtung unempfindlich gegen Verschleiß und Verschmutzung sein soll und das Handling der Vorrichtung und des Meßverfahrens einfach sein soll.

Diese Aufgabe wird mit einer Meßvorrichtung mit den Merkmalen des Anspruches 1 und einem Meßverfahren mit den Merkmalen des Anspruches 10 gelöst. Die jeweiligen Unteransprüche sind auf bevorzugte Ausführungsformen gerichtet. Anspruch 16 beschreibt ein erfindungsgemäßes Kalibrierverfahren für eine erfindungsgemä-ße Meßvorrichtung.

Bei der erfindungsgemäßen Meßvorrichtung ist zumindest eine Abstandssensoreinheit vorgesehen, die derart zu der Meßkammer angeordnet ist, daß sich der gemessene Abstand relativ mit der Ausdehnung der Meßkammer ändert. Mit einer solchen Abstandssensoreinheit läßt sich während der Messung einer Meßgröße, die von der Ausdehnung der Meßkammer abhängt, ein Abstand bestimmen, der von der Ausdehnung der Meßkammer abhängt. Die Meßgröße gestattet bei bekannter Ausdehnung der Meßkammer in Meßrichtung die Bestimmung des interessierenden medizinischen Parameters. Mit der erfindungsgemäßen Vorrichtung ist es also möglich, während der Behandlung nicht nur die abstandsabhängige Meßgröße zu bestimmen, sondern auch den Abstand bzw. dessen Änderung selbst. So läßt sich aus diesen Werten der gewünschte Parameter direkt bestimmen und z. B. auf einer entsprechenden Anzeigeeinheit darstellen und überwachen. Selbstverständlich kann sowohl der so bestimmte Parameter als auch mit ihm in fester Beziehung stehende andere Werte direkt zur Steuerung der Behandlung eingesetzt werden, z. B. zur Änderung der Temperatur, der Flußgeschwindigkeit oder anderen bei der Dialysebehandlung relevanten Parametern.

Bei Beginn der Behandlung, z. B. einer Dialysebehandlung, muß nur die Disposable-Kassette angeschlossen und verschlossen werden. Es muß keine besondere Vorsicht eingesetzt werden, um den Abstand der zwei Elemente der Aufnahmekammer für die Disposable-Kassette genau einzustellen, da während der Behandlung das Signal der Abstandssensoreinheit aufgenommen wird und daraus präzise die Ausdehnung der Meßkammer in der Meßrichtung des Meßelementes bestimmt werden kann.

Toleranzschwankungen der Disposableaufnahme während der Behandlung z. B. aufgrund unterschiedlicher Flüssigkeitsdruckwerte oder Temperaturänderungen werden automatisch berücksichtigt und erfordern keine gesonderte Korrektur.

Die Abstandssensoreinheit kann sich dabei direkt im Bereich der Meßkammer befinden. Auf diese Weise kann direkt die Ausdehnung der Meßkammer in Meßrichtung bestimmt werden, ohne daß zusätzlich das gemessene Abstandssignal in die Ausdehnung der Meßkammer umgerechnet werden müßte. Bei anderen Anordnungen kann es vorteilhaft sein, wenn sich die Abstandssensoreinheit gegenüber der Meßkammer versetzt befindet. So läßt sich der Abstand zwischen den zwei Elementen ebenfalls bestimmen, jedoch bleibt die Messung von der Flüssigkeit, die durch die Meßvorrichtung geschickt wird, unbeeinflußt.

Bei einer vorteilhaften Ausführungsform umfaßt die Abstandssensoreinheit zwei sich nicht berührende Teile, die derart zu der Meßkammer angeordnet sind, daß sich ihr relativer Abstand mit der Ausdehnung der Meßkammer ändert.

Die Abstandssensoreinheit kann z. B. eine kapazitive Einheit sein, wobei die zwei Teile die Elektroden eines Kondensators umfassen. Eine Änderung des Abstandes der Kondensatorplatten ändert die Kapazität, die in bekannter Weise bestimmt werden kann. Ebenso kann eine optische Abstandsmessung vorgesehen sein.

Bei einer besonders vorteilhaften Ausführungsform umfaßt die Abstandssensoreinheit zumindest eine Einrichtung zur Erzeugung eines elektrischen und/oder magnetischen Feldes und zumindest einen Feldsensor zur Messung des Feldes bzw. dessen Änderung. Ändert sich der Abstand zwischen der Felderzeugungseinrichtung und dem Feldsensor, so ändert sich auch das Feld, z. B. in seiner Stärke und/oder Richtung. Aus dieser Feldänderung kann auf die Abstandsänderung zurückgeschlossen werden. Bei geeigneter Kalibrierung läßt sich der Abstand auch absolut bestimmen.

Wird ein Magnetfeld zur Abstandsmessung eingesetzt, so kann die Änderung des Abstandes z. B. durch Messung von induzierten Wirbelströmen bestimmt werden.

Besonders einfach zu realisieren ist jedoch die Magnetfeldmessung mit Hilfe eines magnetoresistiven Sensors, wie er z. B. in DE 197 46 199 beschrieben ist. Bei einem solchen magnetoresistiven Sensor ändert sich in gut messbarer Weise der Widerstand einer Sensoranordnung bei Änderung des ihn durchdringenden magnetischen Feldes. Befindet sich nun z. B. auf einer Seite der Disposable-Kassette ein ein Magnetfeld erzeugendes Element, einfacherweise z. B. ein Permanentmagnet, und auf der anderen Seite der Disposable-Kassette ein magnetoresistiver Sensor, so ist das Signal des magnetoresistiven Sensors vom Abstand abhängig, der wiederum direkt mit der Ausdehnung der Meßkammer zusammenhängt. Das elektrische Signal des magnetoresistiven Sensors läßt sich einfach messen und präzise auswerten. Bei einem Sensor gemäß DE 197 46 199 wird mit Hilfe eines Sensors die Richtung des Magnetfeldes bezüglich der Sensorachse erfaßt. Die erfindungsgemäße Anordnung wird dann derart gewählt, daß die Richtung des Magnetfeldes am Ort des Feldsensors eindeutig mit dem Abstand des Feldsensors von der Felderzeugungseinrichtung zusammenhängt, um so den Abstand aus der Richtung des Magnetfeldes zu bestimmen.

Bei der Dialysebehandlung ist die genaue Kenntnis des Wassergehaltes und/oder der damit zusammenhängenden Blutdichte notwendig. Die Dichtemessung kann z. B. eine Lichtabsorptionsmessung umfassen. Besonders präzise ist jedoch die Messung der Laufzeit einer Ultraschallwelle durch das Blut, deren Schallgeschwindigkeit abhängig von der Dichte ist. Aus der Laufzeit läßt sich so auch auf den Hämatokritwert zurückschließen. Für die Ultraschallmessung kann ein Ultraschallsensor auf einer Seite der Meßkammer und ein Ultraschallempfänger auf der gegenüberliegenden Seite der Meßkammer vorgesehen sein. Selbstverständlich kann auch auf einer Seite der Meßkammer der Sensor und der Empfänger vorgesehen sein und auf der anderen Seite nur ein Ultraschallreflektor.

Mit einer solchen Ausgestaltung der Meßvorrichtung läßt sich als Meßgröße die Laufzeit der Ultraschallwelle bestimmen. Aus dieser Meßgröße läßt sich bei bekanntem Abstand der Ultraschallmeßelemente der gewünschte medizinische Parameter bestimmen, wobei mit der erfindungsgemäßen Meßvorrichtung die Bestimmung dieses Abstandes oder dessen Änderung präzise möglich ist. Selbstverständlich kann so auch die Ultraschallgeschwindigkeit selbst bestimmt werden.

Mit Hilfe einer Abstandssensoreinheit läßt sich an einer Stelle der Disposable-Kassette der Abstand überwachen und so auf eine Änderung der Ausdehnung der Meßkammer in der Meßrichtung zurückschließen. Eine höhere Präzision läßt sich erreichen, wenn an mehreren, seitlich gegeneinander versetzten Stellen der Meßvorrichtung Abstandssensoreinheiten vorgesehen sind. Auf diese Weise läßt sich auch eine Verkippung oder Versetzung der verschiedenen Teile der Meßvorrichtung detektieren.

Bei einem erfindungsgemäßen Verfahren zur Bestimmung von Parametern medizinischer Flüssigkeiten wird während der Bestimmung einer Meßgröße, deren Wert von der Ausdehnung einer Meßkammer abhängt, ein zur Ausdehnung der Meßkammer in der Meßrichtung bzw. der Änderung der Ausdehnung in fester Beziehung stehender Wert bestimmt. Besonders vorteilhaft wird bei dem erfindungsgemäßen Verfahren ein magnetisches Feld zur Bestimmung des Abstandes eines Feldsensors und einer Felderzeugungseinrichtung bestimmt, um aus diesem Abstand die Ausdehnung der Meßkammer bestimmen zu können. Dazu kann, wie beschrieben, z. B. eine Richtungsbestimmung des Magnetfeldes eingesetzt werden, wenn die Richtung des Magnetfeldes am Ort des Feldsensors vom Abstand des Feldsensors von der Felderzeugungseinrichtung abhängt.

Mit der erfindungsgemäßen Meßvorrichtung und dem erfindungsgemäßen Meßverfahren ist eine präzise Überwachung der Ausdehnung einer Meßkammer z. B. in einer Disposable-Kassette bei der Dialysebehandlung möglich. Parameter, die aus einer Meßgröße bestimmt werden, die abhängig von der Ausdehnung der Meßkammer ist, lassen sich so präzise und einfach bestimmen. Die Meßvorrichtung ist unempfindlich gegen Verschmutzung und Verschleiß und kann einfach bedient werden. Eine besonders präzise Einstellung oder gesonderte Überwachung des Abstandes ist nicht notwendig.

Die erfindungsgemäße Meßvorrichtung und das erfindungsgemäße Meßverfahren werden anhand der anliegenden Figuren im Detail erläutert, die bevorzugte Ausgestaltungen darstellen. Dabei zeigt
- Figur 1: eine seitliche Schnittansicht einer Ausführungsform einer erfindungsgemäßen Meßvorrichtung mit eingesetzter Disposable-Kassette,
- Figur 2: schematisch ein Detail der Figur 1, und
- Figur 3: eine Schnittansicht auf eine Disposable-Kassette mit angedeuteten Meßeinrichtungen.

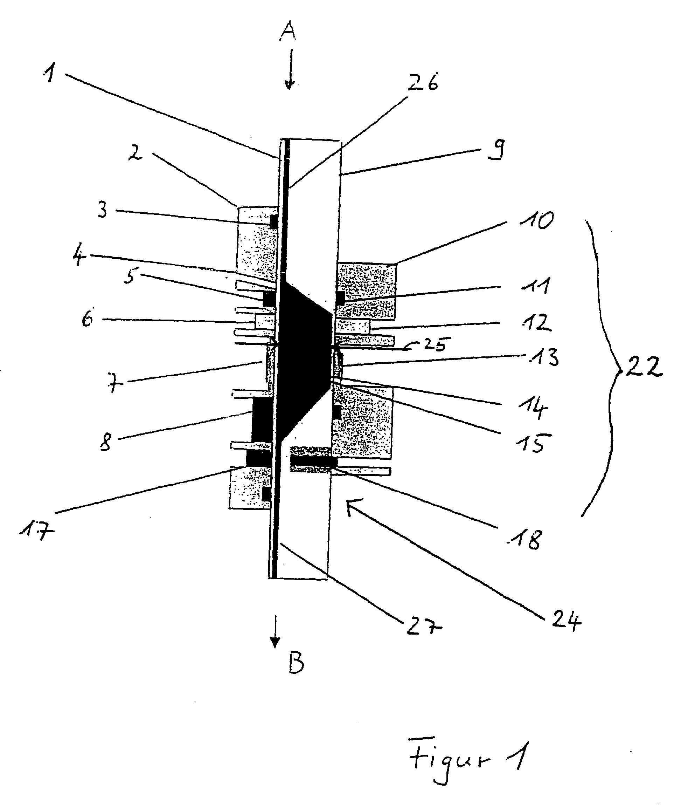

Figur 1 zeigt die erfindungsgemäße Meßvorrichtung mit einer eingesetzten Disposable-Kassette 9. Die Disposable-Kassette ist in einer Aufnahmekammer z. B. einer Dialysemaschine eingesetzt. In dem Disposable 9 ist eine Meßkammer 15 integriert, die einen Zufluß 26 und einen Abfluß 27 aufweist und eine Tiefe 25 hat. An jeweils gegenüberliegenden Seiten ist die Meßkammer 15 mit einer Folie 1, 14 abgeschlossen. Beidseitig der Meßkammer liegen an den Folien 1, 14 jeweils Sensorträger 2, 10 an, die an ihrem äußeren Rand jeweils eine Dichtung 3, 11 aufweisen. Die Dichtung ist dabei in einer jeweils dafür vorgesehenen Nut eingesetzt und ragt geringfügig über die Unterseite der Sensorträger hinaus. Figur 1 zeigt dabei den Meßbereich 22 der Disposable-Kassette zum Einsatz bei einer Dialysebehandlung. Die Sensorträger sind Teil der Dialysemaschine.

Mit 5 ist ein Temperatursensor bezeichnet, der die Messung der Temperatur von Blut in der Meßkammer 15 erlaubt. 6 und 12 bezeichnen Empfänger und Sender einer Lichtschranke zur Feststellung, ob Blut durch die Meßkammer fließt. 8 bezeichnet einen Drucksensor, der derart an der Folie 1 anliegt, die die Meßkammer 15 abschließt, daß der Druck des Blutes bestimmt werden kann. 7, 13 bezeichnet ein Meßelement, im gezeigten Fall einen Ultraschallempfänger 7 und einen Ultraschallsender 13, die beidseitig der Meßkammer angeordnet sind. Beispielhaft bezeichnet 4 einen Anschluß für Unterdruck. 24 bezeichnet eine Abstandssensoreinheit bestehend aus zwei Teilen 17 und 18. Die einzelnen Sensoren 5, 6, 12, 7, 13, 8, 24 sind in nicht gezeigter Weise mit Anzeigeeinheiten oder einer Steuerungseinheit, z. B. einem Mikroprozessor, verbunden.

A und B bezeichnen die Strömungsrichtung des Blutes während der Dialysebehandlung durch die Disposable-Kassette.

Figur 2 zeigt die Abstandssensoreinheit 24 schematisch vergrößert im Detail. Im Sensorträger 2 befindet sich ein erster Magnet 18 mit der beispielhaften Anordnung von Nord- und Südpol. In dem anderen Sensorträger 10 auf der anderen Seite der Kassette befindet sich der zweite Teil 17 der Abstandssensoreinheit 24. Dieser Teil umfaßt einen zweiten Magneten 19 und einen magnetoresistiven Sensor 20. Schematisch sind Anschlüsse 21 angedeutet. Wiederum nur beispielhaft sind die Pole Nord- und Südpol des zweiten Magneten 19 angedeutet. Der Abstand zwischen den zwei Teilen 17 und 18 der Abstandssensoreinheit 24, der überwacht werden soll, ist mit 23 bezeichnet. Bei der Anordnung der Figur 2 wird durch den Magnet 18 ein Magnetfeld am Ort des Sensors 20 erzeugt, dessen Feldlinien nahezu horizontal, im gezeigten Beispiel von rechts nach links, verlaufen. Das Magnetfeld des zweiten Magneten 19 führt dagegen zu einem Magnetfeld am Ort des Sensors mit einer dominanten vertikalen Komponente (hier von unten nach oben). Das resultierende Magnetfeld hat je nach Abstand 23 der beiden Magneten 18, 19 eine unterschiedliche Ausrichtung in der Zeichenebene. Ist der Abstand 23 groß, so entspricht das Magnetfeld eher dem des zweiten Magneten 19. Ist der Abstand klein, so kippt bei der gezeigten Anordnung der Feldvektor nach links, da sich eine horizontale Komponente in den Feldvektor mischt. Damit sich eine nennenswerte Drehung ergibt, ist es je nach Anordnung relativ zum Sensor sinnvoll, wie in Figur 2 beide Magnete gedreht zueinander anzuordnen. Mit einer solchen Anordnung kann der Feldwinkel zu einer Sensorachse und somit die Richtung des Magnetfeldes gemessen werden, die mit dem Abstand direkt korelliert ist.

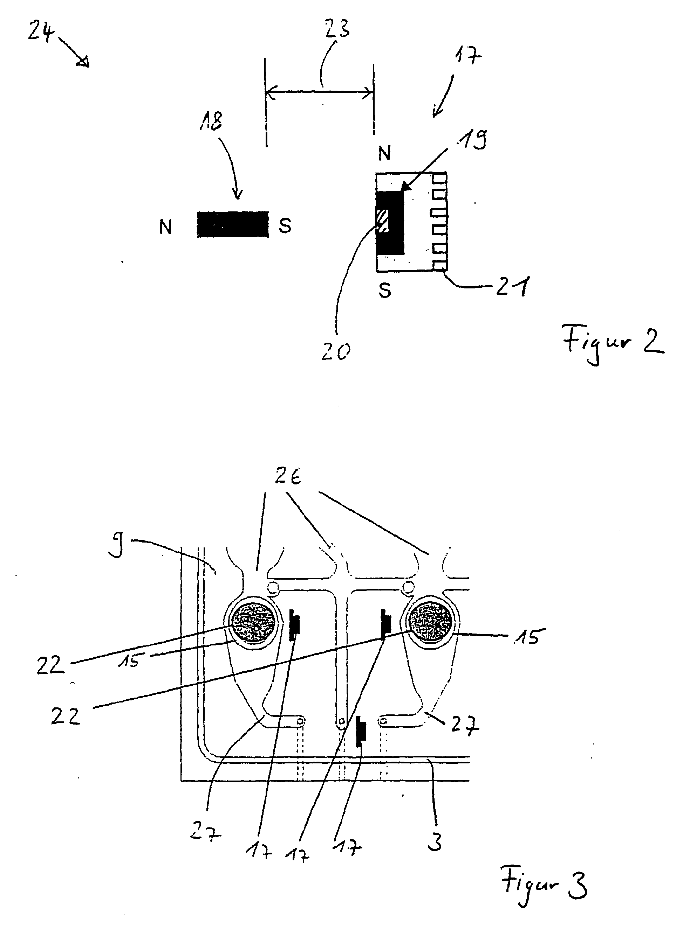

In Figur 3 ist eine Disposable-Kassette im Schnitt gezeigt. Sichtbar ist das Disposable 9 mit den darin eingelassenen Kanälen 26 für den Zufluß und 27 für den Abfluß des Blutes. In Figur 3 ist dabei die Disposable-Kassette zusammen mit der gedachten Position der dahinter liegenden Sensorteile 17 der Abstandssensoreinheit 24 gezeigt, die hinter dem Disposable 9 angeordnet sind, wenn die Kassette in die Dialysemaschine eingelegt ist. Weiterhin sind beim gezeigten Beispiel zwei Meßbereiche 22 schematisch dargestellt. Ist die Disposable-Kassette 9 in die entsprechende Aufnahmekammer der Dialysemaschine eingelegt, so befinden sich in diesen Bereichen 22, wie in Figur 1 dargestellt, die Sensoren, wie z. B. der optische Sensor 6, 12, der Temperatursensor 5 oder der Drucksensor 8. Weiterhin weisen die Meßbereiche 22 z. B. den Ultraschallaufzeitmeßsensor 7, 13 auf.

Die Meßvorrichtung wird vorzugsweise zur Dialysebehandlung eingesetzt. Hierzu wird nach Einlegen der Disposable-Kassette die Meßvorrichtung in den extrakorporalen Blutkreislauf geschaltet. Das Blut fließt in Richtung der Pfeile A, B.

Vor den einzelnen Messungen wird zunächst an die Folien Unterdruck angelegt, z. B. über den Anschluß 4 oder vergleichbare Anschlüsse, so daß die Folien 1, 14 in gutem Kontakt zu den Sensoren stehen. Die Sensoren können über eine nicht näher dargestellte Steuereinheit angesteuert werden, so daß die jeweiligen Messungen beginnen können.

Zur Messung der Blutdichte bzw. des Hämatokritwertes wird mit Hilfe des Ultraschallsenders 13 eine Ultraschallwelle durch die Meßkammer 15 geschickt und vom Ultraschallempfänger 7 empfangen. Die dabei gemessene Meßgröße ist die Laufzeit der Ultraschallwelle, die von der Schallgeschwindigkeit abhängt, die wiederum vom Hämatokritwert bzw. der Dichte des Blutes abhängig ist. Mit Hilfe der Abstandssensoreinheit 24 wird der Abstand zwischen den Teilen 17, 18 der Abstandssensoreinheit durch Auslesen des magnetoresistiven Sensors 20 bestimmt.

Dieser Abstand hängt in fester Beziehung mit dem Abstand der Sensorträger 2, 10 zusammen und läßt auf diese Weise die exakte Bestimmung des Abstandes des Ultraschallsenders 13 und des Ultraschallempfängers 7 zu. Mit Hilfe der gemessenen Laufzeit und dem auf diese Weise bestimmten Abstand, der der Ausdehnung 25 der Meßkammer 15 entspricht, kann aus der Laufzeit der Ultraschallwelle der gewünschte Parameter bestimmt werden (z. B. die Schallgeschwindigkeit und damit die Zusammensetzung des Blutes), der dann ggf. überwacht und ggf. zur Steuerung der Behandlung eingesetzt werden kann.

Durch die genaue Abstandsbestimmung bzw. dessen Änderung mit Hilfe der Abstandssensoreinheit 24 ist eine genaue Justierung der einzelnen Sensorträger 2, 10 nicht notwendig. Bei einer Ausführungsform gemäß der Figur 3 ist auch eine Verkippung oder Versetzung aus den unterschiedlichen Signalen der einzelnen gegeneinander versetzten Abstandssensoreinheiten feststellbar.

Der magnetoresistive Abstandssensor kann wie folgt kalibriert werden. Dabei kann die Ultraschallmessungseinrichtung 7, 13 verwendet werden. Bereits beim Bau der Dialysemaschine wird werkseitig mit Hilfe eines Disposables bekannter Eigenschaften und einer Flüssigkeit bekannter Eigenschaften, wie z. B. Kochsalzlösung, bei definiertem Abstand die Ultraschallaufzeit t1 bestimmt, die der Schall von den Ultraschallsensorteilen 7, 13 bis zur Flüssigkeit benötigt. Diese Zeit t1 umfaßt zum einen die Zeit, die der Schall vom Sender 13 bis an das Disposable 9 und vom Disposable 9 bis zum Empfänger 7 benötigt. Zum anderen geht in diese Schallaufzeit t1 diejenige Zeit ein, die der Schall sich in denjenigen Teilen des Disposables 9 selbst bewegt, die zwischen den Sensoren 7, 13 angeordnet sind. Dies sind beim gezeigten Ausführungsbeispiel der Figur 1 z. B. die Folien 1 und 14.

Vor einer Behandlung wird zur Kalibrierung wiederum das Disposable 9 mit einer bekannten Flüssigkeit, z. B. Kochsalzlösung, durchflossen. Der Temperatursensor 5 kann zur Temperaturkorrektur der Schallaufzeit herangezogen werden. Es wird bei eingesetztem Disposable die Gesamtlaufzeit t des Ultraschalles vom Sender 13 zum Empfänger 7 gemessen. Diese setzt sich zusammen aus der bekannten Zeit t1 und der Laufzeit t2, die auf den Schallweg in der Kochsalzlösung in der Meßkammer 15 entfällt. So kann t2 aus der gemessenen Laufzeit t und der bekannten Schallaufzeit t1 bestimmt werden. Die temperaturabhängige Ausbreitungsgeschwindigkeit in der Kochsalzlösung ist bekannt, wodurch der Abstand 25, der auf den Meßweg innerhalb der Flüssigkeit entfällt, bestimmt werden kann. Im Anschluß wird der Abstand zwischen den Sensorplatten 2, 10 ein wenig vergrößert bzw. verkleinert. Dies kann z. B. mit einer hier nicht weiter interessierenden Vorrichtung geschehen, mit der eine der Sensorplatten bewegt werden kann.

Dabei wird jeweils der berechnete Abstand sowie das Meßsignal des magnetoresistiven Sensors aufgenommen und abgespeichert. So erhält man eine Kennlinie, die mit Hilfe des magnetoresistiven Sensors eine Abstandsmessung bei unbekannter Flüssigkeit erlaubt. Eine solche Kalibrierung gestattet die Erfassung von Abstandsänderungen in der Größenordnung von 1 µm.

## Patentansprüche

1. Meßvorrichtung zur Bestimmung von Parametern medizinischer Flüssigkeiten mit einem Meßbereich, der folgendes umfaßt:
- eine Meßkammereinrichtung zur Aufnahme einer Disposable-Kassette (9) mit zumindest einer Meßkammer (15), durch die die zu messende Flüssigkeit geführt wird,
- zumindest ein Meßelement (13, 7) zur Messung einer Meßgröße, deren Wert von der Ausdehnung der Meßkammer (15) in einer Meßrichtung abhängt und bei bekannter Ausdehnung der Meßkammer (15) die Bestimmung des Parameters oder eines dazu in fester Beziehung stehenden Wertes zuläßt,
**gekennzeichnet durch**
zumindest eine Abstandssensoreinheit (24), die derart zu der Meßkammereinrichtung angeordnet ist, daß sich der von der Abstandssensoreinheit gemessene Abstand (23) relativ mit der Ausdehnung (25) der Meßkammer (15) ändert.

2. Meßvorrichtung nach Anspruch 1, **dadurch gekennzeichnet, daß** die zumindest eine Abstandssensoreinheit (24) zumindest zwei sich nicht berührende Teile (17, 18) umfaßt, die derart zu der Meßkammereinrichtung angeordnet sind, daß sich ihr relativer Abstand (23) mit der Ausdehnung (25) der Meßkammer (15) ändert.

3. Meßvorrichtung nach einem der Ansprüche 1 oder 2, **dadurch gekennzeichnet, daß** die Abstandssensoreinheit (24) zumindest eine Einrichtung (18, 19) zur Erzeugung eines elektrischen und/oder magnetischen Feldes und zumindest einen Sensor (20) zur Messung des erzeugten magnetischen und/oder elektrischen Feldes oder dessen Änderung umfaßt.

4. Meßvorrichtung nach Anspruch 3, **dadurch gekennzeichnet, daß** die Felderzeugungseinrichtung eine Einrichtung zur Erzeugung eines Magnetfeldes, vorzugsweise zumindest einen Permanentmagneten (18), umfaßt und der Feldsensor einen magnetoresistiven Sensor (20) umfaßt.

5. Meßvorrichtung nach einem der Ansprüche 1 bis 4, **dadurch gekennzeichnet, daß** das Meßelement einen Ultraschallsender (13) und einen Ultraschallempfänger (7) umfaßt, die derart bezüglich einer in der Meßkammereinrichtung aufgenommenen Meßkammer (15) angeordnet sind, daß sie eine Ultraschallaufzeitmessung durch die Meßkammer erlauben.

6. Meßvorrichtung nach einem der Ansprüche 1 bis 5, **dadurch gekennzeichnet, daß** die Vorrichtung zum Einsatz bei der Dialyse und zur Bestimmung von Parametern von Blut geeignet ist.

7. Meßvorrichtung nach einem der Ansprüche 1 bis 6, **dadurch gekennzeichnet, daß** die Vorrichtung zur Bestimmung der Dichte und/oder der Konzentration und/oder des Hämatokritwertes der medizinischen Flüssigkeit geeignet ist.

8. Meßvorrichtung nach einem der Ansprüche 1 bis 7, **dadurch gekennzeichnet, daß** die Abstandssensoreinheit (24) gegenüber einer in der Meßkammereinrichtung aufgenommenen Meßkammer (15) seitlich versetzt angeordnet ist.

9. Meßvorrichtung nach einem der Ansprüche 1 bis 8, **gekennzeichnet durch** zumindest zwei Abstandssensoreinheiten (24), die versetzt zueinander angeordnet sind.

10. Verfahren zur Bestimmung von Parametern medizinischer Flüssigkeiten, bei dem
- die Flüssigkeit durch eine Meßkammer (15) geführt wird,
- eine Meßgröße gemessen wird, deren Wert von der Ausdehnung der Meßkammer (15) in einer Meßrichtung abhängt und bei bekannter Ausdehnung der Meßkammer (15) die Bestimmung des Parameters oder eines dazu in fester Beziehung stehenden Wertes zuläßt,
**dadurch gekennzeichnet, daß**
bei der Messung der Meßgröße zusätzlich ein zu der Ausdehnung (25) der Meßkammer (15) in der Meßrichtung oder der Änderung der Ausdehnung in fester Beziehung stehender Wert bestimmt wird.

11. Verfahren nach Anspruch 10, **dadurch gekennzeichnet, daß** die Ausdehnung (25) mit Hilfe eines elektrischen und/oder magnetischen Feldes bestimmt wird, das von einer Felderzeugungseinrichtung (18) am Ort eines Feldsensors (20) erzeugt wird, die derart angeordnet sind, daß ihr Abstand (23) in fester Beziehung zur Ausdehnung (25) steht.

12. Verfahren nach Anspruch 11, bei dem ein Magnetfeld mit einerMagnetfelderzeugungseinrichtung, vorzugsweise zumindest einem Permanentmagneten (18), erzeugt wird und seine Stärke und/oder Richtung mit einem magnetoresistiven Sensor (20) gemessen wird.

13. Verfahren nach Anspruch 10, **dadurch gekennzeichnet, daß** die Änderung der Ausdehnung (25) aus der Änderung eines elektrischen und/oder magnetischen Feldes bestimmt wird, die sich aus der Änderung eines Abstandes (23) zwischen einer Felderzeugungseinrichtung (18) und einem Feldsensor (20) ergibt.

14. Verfahren nach einem der Ansprüche 10 bis 13, bei dem die Meßgröße die Laufzeit einer Ultraschallwelle umfaßt.

15. Verfahren nach einem der Ansprüche 10 bis 14, bei dem der zu bestimmende Parameter die Dichte und/oder die Konzentration und/oder der Hämatokritwert ist.

16. Verfahren zur Kalibrierung einer Meßvorrichtung nach einem der Ansprüche 1 bis 9, bei dem
a) eine Flüssigkeit mit einem bekannten Wert eines Parameters durch die Meßkammer (15) geschickt wird, zu dessen Bestimmung die Meßvorrichtung geeignet ist,
b) mit dem Meßelement (7, 13) eine Meßgröße gemessen wird, deren Wert von der Ausdehnung (2) der Meßkammer (15) in der Meßrichtung abhängt,
c) die Ausdehnung (25) der Meßkammer (15) in der Meßrichtung aus dem bekannten Parameter und der gemessenen Meßgröße oder einem dazu in fester Beziehung stehenden Wert bestimmt wird,
d) ein Signal der Abstandssensoreinheit (17, 18) gemessen wird,
e) das gemessene Signal dem bestimmten Wert der Ausdehnung (25) der Meßkammer (15) oder einem dazu in fester Beziehung stehenden Wert zugeordnet wird,
f) die Ausdehnung (25) der Meßkammer (15) geändert wird, und
g) die Schritte b) bis f) mindestens einmal zur Bestimmung einer Kennlinie zwischen dem Signal der Abstandssensoreinheit (17, 18) und der Ausdehnung (25) der Meßkammer (15) bzw. dem dazu in fester Beziehung stehenden Wert wiederholt werden.

17. Verfahren zur Kalibrierung nach Anspruch 16, bei dem der Parameter die Ultraschallgeschwindigkeit ist, das Meßelement einen Ultraschallsender (13) und einen Ultraschallempfänger (7) umfaßt und die Meßgröße die Ultraschallaufzeit ist.

18. Verfahren zur Kalibrierung nach Anspruch 17, bei dem als Flüssigkeit Kochsalzlösung verwendet wird.

## Claims

1. A measuring apparatus for determining parameters of medical fluids with a measuring region which includes the following:
- a measuring chamber means for receiving a disposable cassette (9) with at least one measuring chamber (15), through which the fluid to be measured is guided, and
- at least one measuring element (13, 7) for measuring a measurement value, the value of which depends on the extent of the measuring chamber (15) in a measuring direction and when the extent of the measuring chamber (15) is known, permits the parameter or a value which is in a fixed relationship therewith to be determined,
**characterised by**
at least one spacing sensor unit (24) which is arranged relative to the measuring chamber means in such a way that the spacing (23) measured by the spacing sensor unit changes relatively with the extent (25) of the measuring chamber (15).

2. A measuring apparatus according to claim 1 **characterised in that** the at least one spacing sensor unit (24) comprises at least two parts (17, 18) which are not in contact with each other and which are arranged relative to the measuring chamber means in such a way that their relative spacing (23) changes with the extent (25) of the measuring chamber (15).

3. A measuring apparatus according to one of claims 1 and 2 **characterised in that** the spacing sensor unit (24) includes at least one device (18, 19) for producing an electrical and/or magnetic field and at least one sensor (20) for measuring the produced magnetic and/or electrical field or the change therein.

4. A measuring apparatus according to claim 3 **characterised in that** the field producing device includes a device for producing a magnetic field, preferably at least one permanent magnet (18), and the field sensor includes a magnetoresistive sensor (20).

5. A measuring apparatus according to one of claims 1 to 4 **characterised in that** the measuring element includes an ultrasonic transmitter (13) and an ultrasonic receiver (7) which are arranged relative to a measuring chamber (15) accommodated in the measuring chamber means in such a way that they allow ultrasound transit time measurement through the measuring chamber.

6. A measuring apparatus according to one of claims 1 to 5 **characterised in that** the apparatus is suitable for use in dialysis and for determining parameters of blood.

7. A measuring apparatus according to one of claims 1 to 6 **characterised in that** the apparatus is suitable for determining the density and/or the concentration and/or the haemocrit value of the medical fluid.

8. A measuring apparatus according to one of claims 1 to 7 **characterised in that** the spacing sensor unit (24) is arranged in laterally displaced relationship with a measuring chamber (15) accommodated in the measuring chamber means.

9. A measuring apparatus according to one of claims 1 to 8 **characterised by** at least two spacing sensor units (24) which are arranged in mutually displaced relationship.

10. A method of determining parameters of medical fluids in which
- the fluid is passed through a measuring chamber (15), and
- a measurement value is measured, the value of which depends on the extent of the measuring chamber (15) in a measuring direction and when the extent of the measuring chamber (15) is known, permits the parameter or a value which is in a fixed relationship therewith to be determined,
**characterised in that**
in measurement of the measurement value, in addition a value which is in a fixed relationship with the extent (25) of the measuring chamber (15) in the measuring direction or the change in extent is determined.

11. A method according to claim 10 **characterised in that** the extent (25) is determined by means of an electrical and/or magnetic field which is produced by a field producing device (18) at the location of a field sensor (20), which are so arranged that their spacing (23) is in a fixed relationship with the extent (25).

12. A method according to claim 11 in which a magnetic field is produced with a magnetic field producing device, preferably at least one permanent magnet (18), and its strength and/or direction is measured with a magnetoresistive sensor (20).

13. A method according to claim 10 **characterised in that** the change in the extent (25) is determined from the change in an electrical and/or magnetic field which arises out of the change in a spacing (23) between a field producing device (18) and a field sensor (20).

14. A method according to one of claims 10 to 13 in which the measurement value includes the transit time of an ultrasonic wave.

15. A method according to one of claims 10 to 14 in which the parameter to be determined is the density and/or the concentration and/or the haemocrit value.

16. A method of calibrating a measuring apparatus according to one of claims 1 to 9 in which
a) a fluid with a known value of a parameter is sent through the measuring chamber (15), the measuring apparatus being suitable for determining same,
b) a measurement value is measured with the measuring element (7, 13), the value thereof depending on the extent (2) of the measuring chamber (15) in the measuring direction,
c) the extent (25) of the measuring chamber (15) in the measuring direction is determined from the known parameter and the measured measurement value or a value which is in a fixed relationship therewith,
d) a signal of the spacing sensor unit (17, 18) is measured,
e) the measured signal is associated with the determined value of the extent (25) of the measuring chamber (15) or a value which is in a fixed relationship therewith,
f) the extent (25) of the measuring chamber (15) is changed, and
g) steps b) to f) are repeated at least once for determining a characteristic curve between the signal of the spacing sensor unit (17, 18) and the extent (25) of the measuring chamber (15) or the value which is in a fixed relationship therewith.

17. A calibration method according to claim 16 in which the parameter is the ultrasound speed, the measuring element includes an ultrasonic transmitter (13) and an ultrasonic receiver (7) and the measurement value is the ultrasound transit time.

18. A calibration method according to claim 17 in which saline solution is used as the fluid.

## Revendications

1. Dispositif de mesure pour déterminer des paramètres de fluides médicaux avec une étendue de mesure, qui comprend les éléments suivants :
- un dispositif de chambre de mesure pour la réception d'une cassette jetable (9) avec au moins une chambre de mesure (15) à travers laquelle le fluide à mesurer est guidé,
- au moins un élément de mesure (13, 7) pour mesurer une grandeur de mesure dont la valeur dépend de la dilatation de la chambre de mesure (15) dans une direction de mesure et, avec une dilatation connue de la chambre de mesure (15), permet la détermination du paramètre ou d'une valeur en relation fixe avec celui-ci,
**caractérisé par**
au moins une unité de capteur de distance (24) qui est disposée par rapport au dispositif de chambre de mesure de telle manière que la distance (23) mesurée par l'unité de capteur de distance varie relativement avec la dilatation (25) de la chambre de mesure (15).

2. Dispositif de mesure selon la revendication 1, **caractérisé en ce que** la au moins une unité de capteur de distance (24) comprend au moins deux parties (17, 18) qui ne sont pas en contact et qui sont placées par rapport au dispositif de chambre de mesure de telle manière que leur distance relative (23) varie avec la dilatation (25) de la chambre de mesure (15).

3. Dispositif de mesure selon l'une quelconque des revendications 1 ou 2, **caractérisé en ce que** l'unité de capteur de distance (24) comprend au moins un dispositif (18, 19) pour générer un champ électrique et/ou magnétique et au moins un capteur (20) pour mesurer le champ électrique et/ou magnétique généré ou sa variation.

4. Dispositif de mesure selon la revendication 3, **caractérisé en ce que** le dispositif de génération de champ comprend un dispositif pour générer un champ magnétique, de préférence au moins un aimant permanent (18), et le capteur de champ comprend un capteur magnéto-résistant (20).

5. Dispositif de mesure selon l'une quelconque des revendications 1 à 4, **caractérisé en ce que** l'élément de mesure comprend un émetteur d'ultrasons (13) et un récepteur d'ultrasons (7) qui sont disposés par rapport à une chambre de mesure (15) logée dans le dispositif de chambre de mesure de telle manière qu'ils permettent une mesure du temps de propagation des ultrasons à travers la chambre de mesure.

6. Dispositif de mesure selon l'une quelconque des revendications 1 à 5, **caractérisé en ce que** le dispositif est adapté pour l'utilisation en dialyse et pour la détermination de paramètres sanguins.

7. Dispositif de mesure selon l'une quelconque des revendications 1 à 6, **caractérisé en ce que** le dispositif est adapté pour la détermination de la densité et/ou de la concentration et/ou de la valeur hématocrite du fluide médical.

8. Dispositif de mesure selon l'une quelconque des revendications 1 à 7, **caractérisé en ce que** l'unité de capteur de distance (24) est décalée latéralement par rapport à une chambre de mesure (15) logée dans le dispositif de chambre de mesure.

9. Dispositif de mesure selon l'une quelconque des revendications 1 à 8, **caractérisé par** au moins deux unités de capteur de distance (24) qui sont décalées l'une par rapport à l'autre.

10. Procédé pour déterminer des paramètres de fluides médicaux selon lequel :
- le fluide est guidé à travers une chambre de mesure (15),
- une grandeur de mesure est mesurée, dont la valeur dépend de la dilatation de la chambre de mesure (15) dans une direction de mesure et, avec une dilatation connue de la chambre de mesure (15), permet la détermination du paramètre ou d'une valeur en relation fixe avec celui-ci,
**caractérisé en ce que**
pendant la mesure de la grandeur de mesure, une valeur en relation fixe avec la dilatation (25) de la chambre de mesure (15) dans la direction de mesure ou avec la variation de la dilatation est déterminée.

11. Procédé selon la revendication 10, **caractérisé en ce que** la dilatation (25) est déterminée à l'aide d'un champ électrique et/ou magnétique qui est généré à l'emplacement d'un capteur de champ (20) par un dispositif de génération de champ (18) qui sont disposés de telle manière que leur distance (23) est en relation fixe avec la dilatation (25).

12. Procédé selon la revendication 11, dans lequel un champ magnétique avec un dispositif de génération de champ magnétique, de préférence au moins un aimant permanent (18), est généré et sa force et/ou sa direction est mesurée à l'aide d'un capteur magnéto-résistant (20).

13. Procédé selon la revendication 10, **caractérisé en ce que** la variation de la dilatation (25) est déterminée à partir de la variation d'un champ électrique et/ou magnétique qui résulte de la variation d'une distance (23) entre un dispositif de génération de champ magnétique (18) et un capteur de champ (20).

14. Procédé selon l'une quelconque des revendications 10 à 13, dans lequel la grandeur de mesure comprend le temps de propagation d'une onde ultrasonore.

15. Procédé selon l'une quelconque des revendications 10 à 14, dans lequel le paramètre à déterminer est la densité et/ou la concentration et/ou la valeur hématocrite.

16. Procédé pour l'étalonnage d'un dispositif de mesure selon l'une quelconque des revendications 1 à 9, dans lequel
a) un fluide ayant une valeur connue d'un paramètre est envoyé à travers la chambre de mesure (15) pour la détermination de laquelle le dispositif de mesure est approprié,
b) une grandeur de mesure, dont la valeur dépend de la dilatation (25) de la chambre de mesure (15) dans la direction de mesure, est mesurée à l'aide de l'élément de mesure (7, 13),
c) la dilatation (25) de la chambre de mesure (15) dans la direction de mesure est déterminée à partir du paramètre connu et de la grandeur de mesure mesurée ou d'une valeur en relation fixe avec ceux-ci,
d) un signal de l'unité de capteur de distance (17, 18) est mesuré,
e) le signal mesuré est associé à la valeur déterminée de la dilatation (25) de la chambre de mesure (15) ou d'une valeur en relation fixe avec celle-ci,
f) on fait varier la dilatation (25) de la chambre de mesure (15), et
g) les étapes b) à f) sont répétées au moins une fois pour déterminer une courbe caractéristique entre le signal de l'unité de capteur de distance (17, 18) et la dilatation (25) de la chambre de mesure (15) ou la valeur en relation fixe avec celle-ci.

17. Procédé pour l'étalonnage selon la revendication 16, dans lequel le paramètre est la vitesse des ultrasons, l'élément de mesure comprend un émetteur d'ultrasons (13) et un récepteur d'ultrasons (7) et la grandeur de mesure est le temps de propagation des ultrasons.

18. Procédé pour l'étalonnage selon la revendication 17, dans lequel on utilise une solution saline comme fluide.
